# EUROPEAN PATENT APPLICATION

(11) **EP 3 488 691 A1**
(43) Date of publication of application: **29.05.2019**
(21) Application number: 18175197.5
(22) Date of filing: 30.05.2018
(51) Int. Cl.: A01M 1/04, A01M 1/20

(54) **CONSTRUCTIVE ARRANGEMENT INTRODUCED INTO FAN HAVING VOLATILIZING FUNCTION OF REPELLENT SUBSTANCE IN A STRIP**

(30) Priority: 27.11.2017 BR 20201725414 U
(71) Applicant: MK Electrodomésticos Mondial S.A., Conceicao do Jacuipe (BR)
(72) Inventor: Krause, Jacques Ivo, Barueri /SP. Brazil. CEP: 06455-000 (BR)
(74) Representative: Negendanck, Matthias

(57) **Abstract**

Whereby a strip (P) containing a substance to be dispersed into the environment is disposed at the front part of the supporting column (1) of the fan (2), beneath the perimeter of its propellers, creating a pressure difference which provides for the propagation of the dispersion of the substance into the environment, and where said supporting column (1) of the fan (2), besides acting as a barrier against air flows produced in the environment, preventing the dilution of the volatilized mist, will also enable the enclosure of the electrical wires (21) used in the connections, making energizing the volatilizing combination much easier, more practical and economical.

## Description

### FIELD OF THE APPLICATION

The present specification pertains to a constructive arrangement introduced into fan having volatilizing function of repellent substance in a strip found on the market, by selective heating provided by the electrical system for driving the fan, and may optionally disperse an air freshener substance also contained in commercial use strips.

The volatilizing combination *per se* is composed of five parts that are easy to assembly which, except for the base, is obtained by encasement, and the movement of the fan propeller, associated to the guidance generated by its front grille, provides for volatilization of the substance contained in the strip in a streamline manner into the environment.

### BACKGROUND OF THE ART

The state of the art already comprises various fan models endowed with means for volatilizing repellent and/or air freshening substances, such as, for example, the development proposed in patent document BRMU8101485-6 pertaining to a cubic body, having a squared horizontal section, endowed at the front with a slot for inserting the air freshening element, also having smaller side slots for inserting strips, forming said slots, outlets for the volatilized substance, and said cubic body being subsequently coupled to the fans and/or circulators; however, the main drawback of this type of embodiment is related to the fact that when in use, part of the substance dispersed adheres to the fan propeller or to the circulator, reducing the substance concentration in the environment, affecting the effectiveness thereof; moreover, due to the fact that said cubic body is situated in the rear part of the fan or of the circulator, the task becomes harder, not only to install and replace the strip, but mainly to verify the operating condition thereof.

Also known in the art is the development proposed in patent document BRMU9000506-6, which discloses a fan endowed with repellent strip support disposed in the central part of its front grille, so as to overcome the drawbacks of the prior arts, associated to the fact that the strip support is installed in the rear part of the fan; however, the main drawback of this type of embodimentis how to install the means of energizing the thermal element to heat the repellent strip, which is fixed in channels located in the support structure, and which passes through the articulable point between said support and the rear grille, and follows on by way of another channel in said rear grille, up to the energizing point of the system, hindering the disassembly and assembly of the front grille, for example, when the need arises to withdraw it for cleaning.

Also known in the art is the development proposed in patent document BRMU8202433-2 which pertains to a fan endowed with electrical device positioned at the rear part, having a receptacle housing the strip repellent or air freshener, and said electrical deviceis topped by a fairing to guide the flow of the volatilized substance; however this type of embodiment has the same drawbacks as already cited, in view of its positioning at the rear part of the fan.

### FUNDAMENTS OF THE MODEL

It is therefore desirable to propose means that enable improved dispersion of the repellent or air freshener substance into the environment, based on simplicity and low cost, and that enables easy visualization of the operating condition of the strip, as well as the replacement thereof.

Therefore, the main objective of the present patent is to propose a constructive arrangement introduced into a fan having a volatilizing function, whereby the strip containing the substance to be dispersed into environment will be disposed at the front part of the supporting column of the fan, immediately beneath the perimeter of its propellers, creating a pressure difference which provides for the propagation of the dispersion of the substance into the environment, and where said supporting column of the fan, besides acting as a barrier against any air flows produced in the environment, preventing dilution of the volatilized mist, will also enable the enclosure of the electrical cables used in the connections, making energizing the volatilizing combination much easier, more practical and economical.

It is a further objective of the present patent to propose the installment of the strip containing the substance to be volatilized at the front part of the supporting column of the fan to facilitate placement and replacement thereof, as well as the visualization of the operating condition.

It is a further objective of the present patent to propose a volatilizing combination comprised by four snap-fit parts at the lower front part of the supporting column of the fan, also having a base, encased, facilitating the manufacturing process thereof.

It is a further objective of the present patent to propose a volatilizing combination which also includes a fifth part, formed by a base, in a single body, which is encased and held, by means of screws, at the lower inner part of the supporting column of the fan.

It is a further objective of the present patent to propose constructive arrangement introduced into a fan having a volatilizing function, in which the heating of said volatilizing combination, guaranteeing the ideal temperature for volatilization of the substance contained in the strip, may vary in intensity according to the increase in rotational speed of the fan propellers, by means of the selective switch thereof.

### SUMMARY OF THE MODEL

The present constructive arrangement introduced into a fan having a volatilizing function incorporates a volatilizing combination of a repellent or air freshener substance contained in the commercial strips designed for such purpose, said volatilizing combination disposed at the front part of the supporting column of the fan, immediately beneath the perimeter of its propellers, with a side entry for installing the strip, and front grille to guide the mist formed by the dispersion of the volatilized substance, which is obtained by heating the thermal plate, energized by the very electrical system of the fan, providing: easy installment, replacement and visualization of the strip; aerodynamic conditions guiding the dispersed mist in relation to the fan propellers, and onwards into the environment without wasting the product.

### BRIEF DESCRIPTION OF THE DRAWINGS

For an improved understanding of the present arrangement introduced into a fan having a volatilizing function, reference is made to the accompanying representative drawings so that it may be fully reproduced using an appropriate technique, enabling its functionality to be characterized, the drawings being merely illustrative, and may have variations, provided that they do not stray from the main function now proposed, wherein:

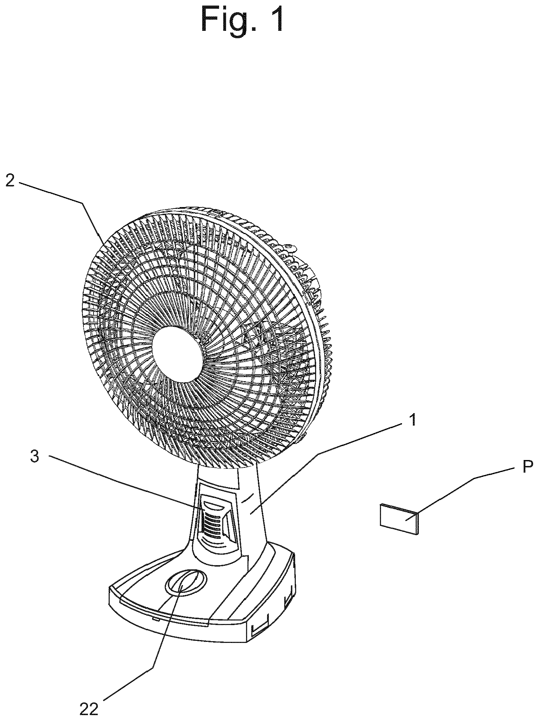

**Figure 1** illustrates a perspective view of the fan, incorporating the volatilizing combination now proposed;

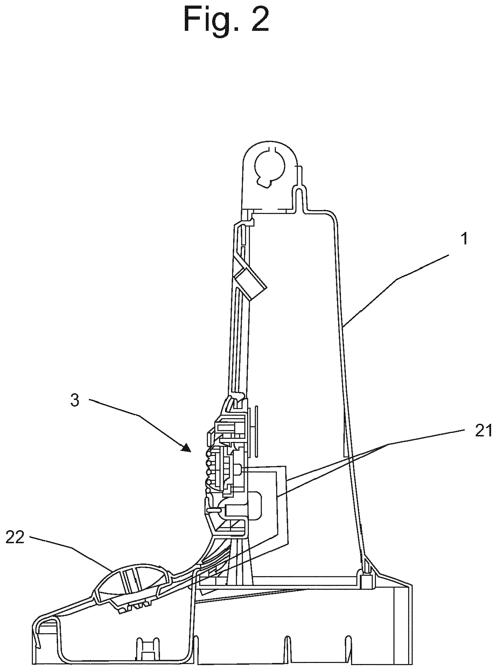

**Figure 2** illustrates a side longitudinal section view of the supporting column of the fan, incorporating the volatilizing combination now proposed;

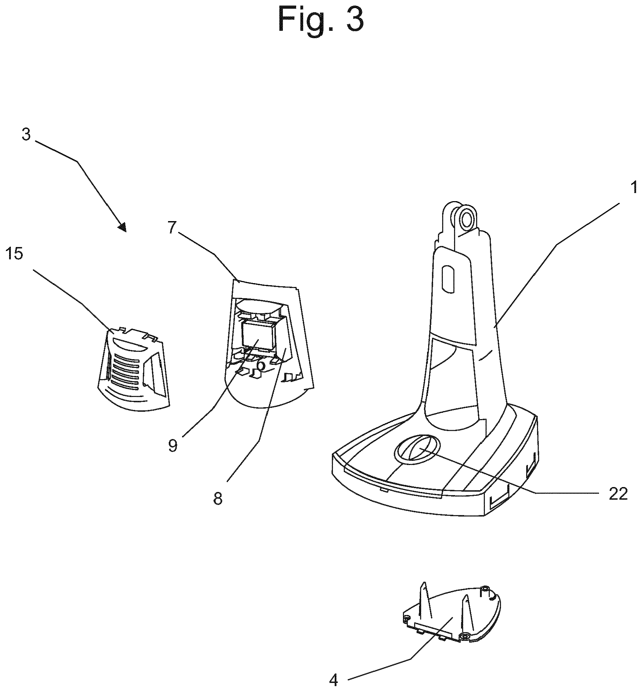

**Figure 3** illustrates an exploded perspective view of the volatilizing combination now proposed in relation to the supporting column of the fan;

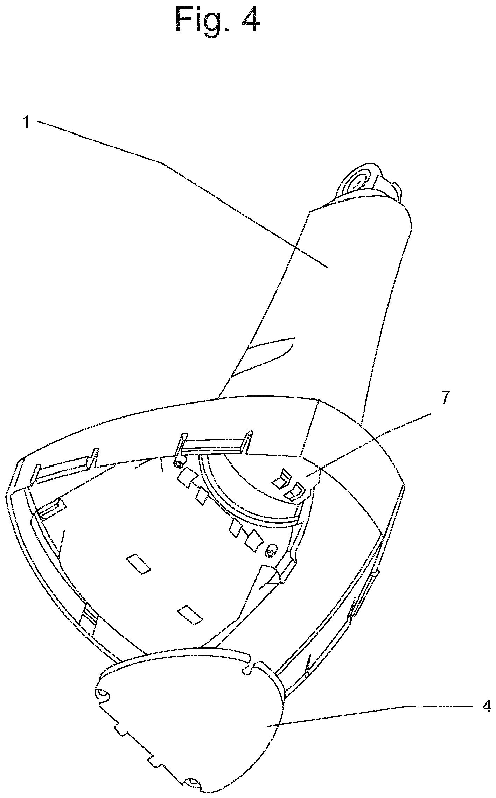

**Figure 4** illustrates an exploded perspective view of the base of the volatilizing combination now proposed in relation to the supporting column of the fan;

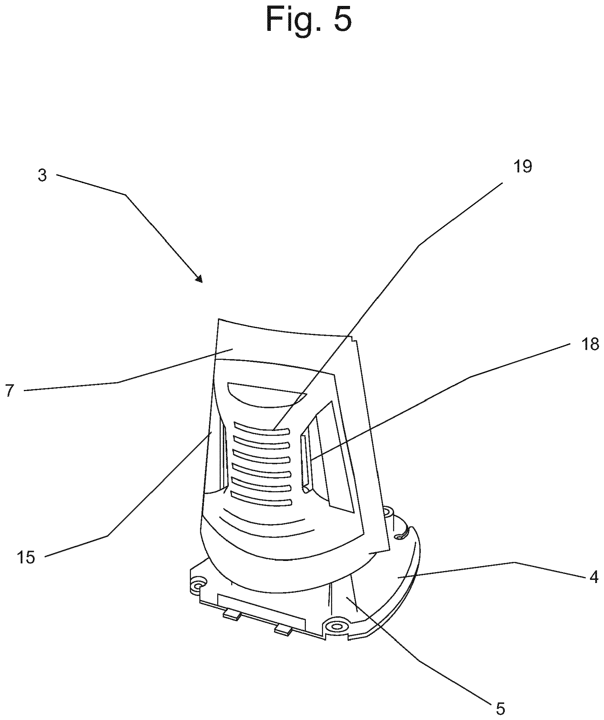

**Figure 5** illustrates a perspective front view of the volatilizing combination now proposed;

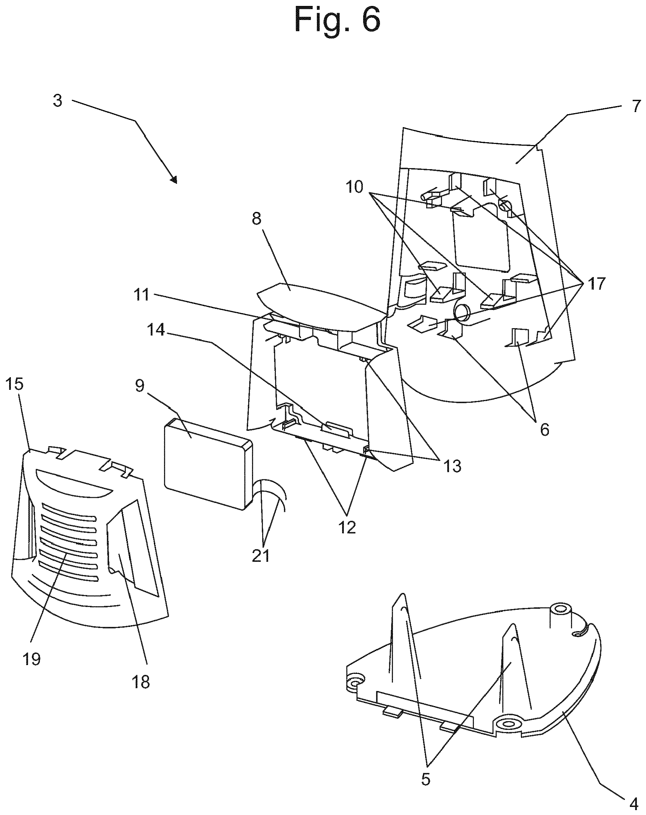

**Figure 6** illustrates an exploded perspective front view of the volatilizing combination now proposed;

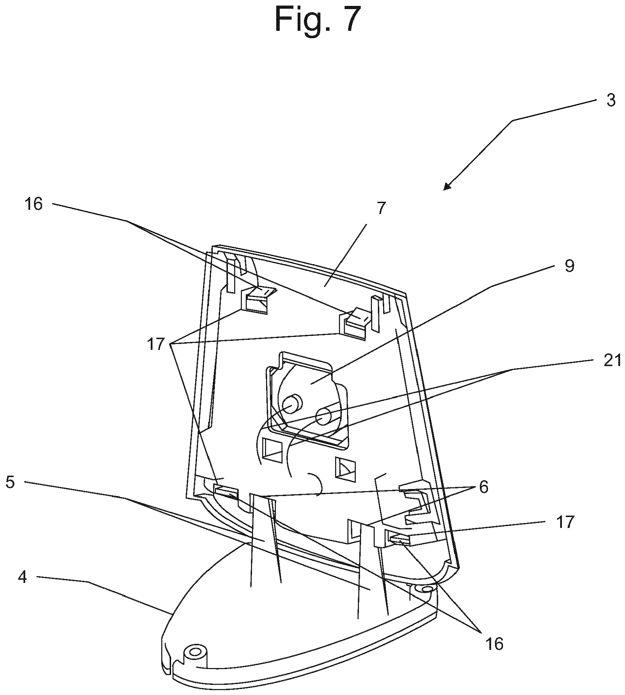

**Figure 7** illustrates a perspective rear view of the volatilizing combination now proposed;

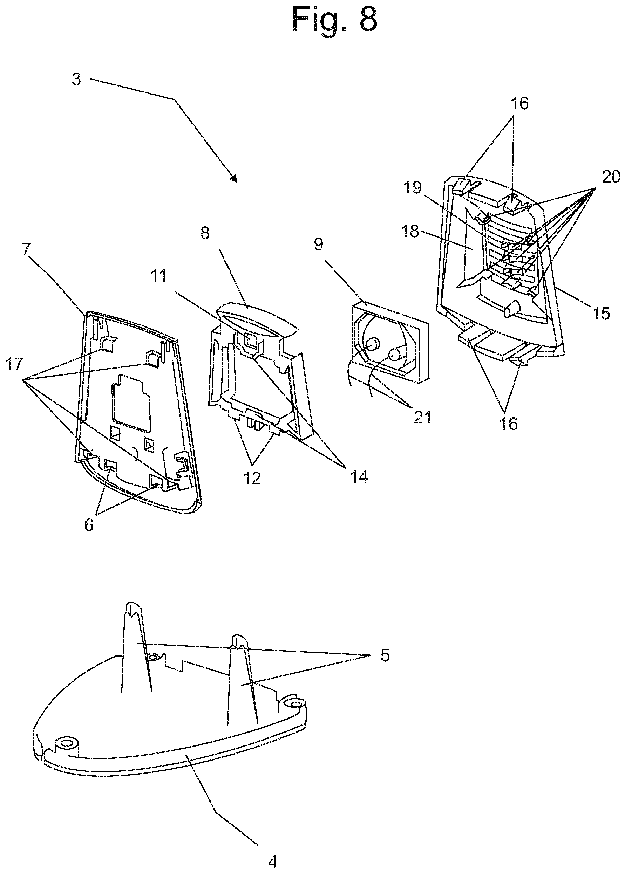

**Figure 8** illustrates an exploded perspective rear view of the volatilizing combination now proposed;

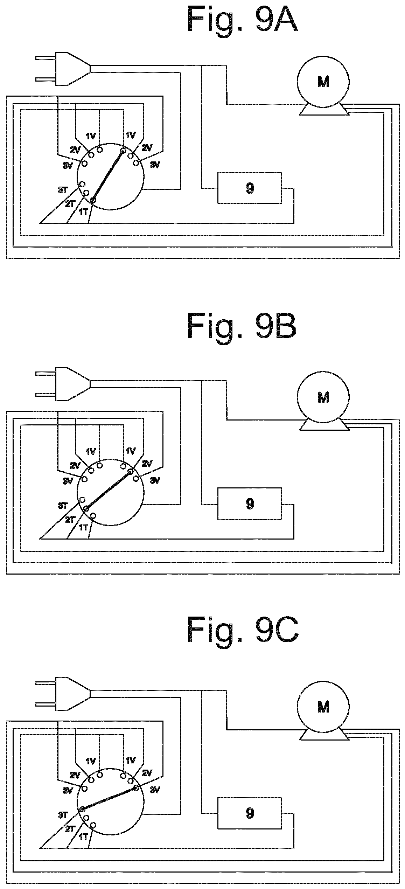

**Figures 9A, 9B and 9C** illustrate the block diagram of the electronic circuit driving the fan speeds, and of the thermal plate of the volatilizing combination now proposed;

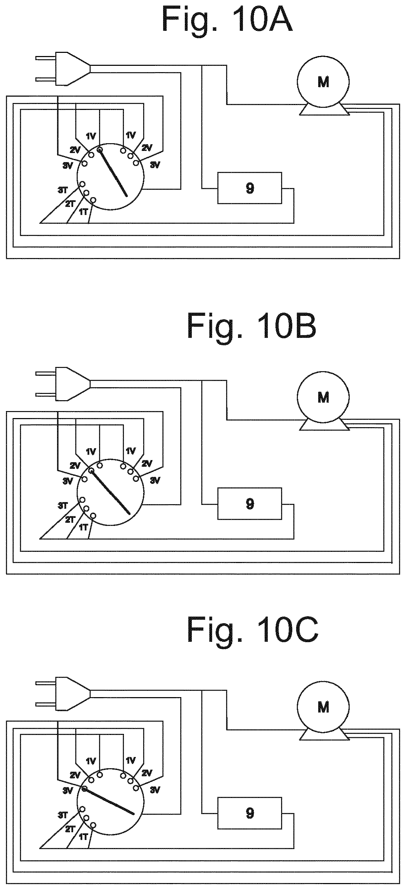

**Figures 10A, 10B and 10C** illustrate the block diagram of the electronic circuit driving the fan speeds, without heating the thermal plate of the volatilizing combination now proposed.

### PREFERRED DESCRIPTION OFTHE MODEL

The present constructive arrangement introduced into a fan having a volatilizing function, now proposed, the installment on the front part of the supporting column (1) of the fan (2), immediately beneath the perimeter of its propellers, volatilizing combination (3) of the substance contained in the strip (P), endowed with a base (4), encased and bolted on the inner part of said column, which besides preventing access to the electrical system of the fan, blocks any air flow which, allowed beneath the supporting column of the fan, might interfere in the dispersion of the volatilized substance and/or its flow towards the front of the fan propellers, having, on the upper surface of the base, orthogonal projections (5), which encase in the openings (6) of the rear support (7), where there is also fixed the frame (8) of the thermal plate (9) to heat the strip, by encasement of tabs (10) of said rear supportin the upper hole (11) and in the lower projections (12) of said frame, and said thermal plate encased between the protrusions (13) provided in the frame, limited at the rear by the upper and lower central protrusions (14), being held in this position by the forward support (15), which is encased by way of the tabs (16) in the openings (17) of the rear support, also having vertical side opening (18) at the front part on the forward support to install and replace the strip, in addition to horizontal slots (19) which guide the mist of the dispersed substance to the region immediately beneath the perimeter of the fan propellers, and the strip, installed through the side openings of the forward support, is positioned in relation to the horizontal slots by the action of distancers (20), provided on the rear surface of the forward support, which also facilitates the replacement thereof, and said slots further prevent contact with the thermal plate, whereby preventing burning.

Said volatilizing combinationis powered through the wires (21) by the electrical system of the fan which, through contacts (1T, 2T and 3T), can employ wires with varied resistances for energizing the thermal plate by switching (22), which when turned clockwise drives the fan propellers at the selected speed (V1, V2, and V3) and when turned anti-clockwise drives only the fan propellers at the selected speed (V1, V2, and V3).

## Claims

1. **A CONSTRUCTIVE ARRANGEMENT INTRODUCED INTO FAN HAVING VOLATILIZING FUNCTION OF REPELLENT SUBSTANCE IN A STRIP,** wherein provides the installment at the lower front part of the supporting column (1) of the fan (2), immediately beneath the perimeter of its propellers, a volatilizing combination (3) of the substance contained in the strip (P), endowed with a base (4), encased and bolted on the inner part of said column, which prevents access to the electrical system of the fan, and blocks the air flow admitted beneath the supporting column of the fan, which might interfere with the dispersion of the volatilized substance and/or its flow towards the front of the fan propellers, having, on the upper surface of the base, orthogonal projections (5), which encase in the openings (6) of the rear support (7), where there is also fixed the frame (8) of the thermal plate (9), to heat the strip, by encasement of tabs (10) of said rear supportin the upper hole (11) and in the lower projections (12) of said frame, said thermal plate being encased between the protrusions (13) provided in the frame, limited at the rear by the upper and lower central protrusions (14), being held in this position by the forward support (15), which is encased by way of the tabs (16) in the openings (17) of the rear support, also having vertical side openings (18) at the front part on the forward support for installing and replacing the strip, in addition to horizontal slots (19), which guide the mist of the dispersed substance to the region immediately beneath the perimeter of the fan propellers, the strip being installed through the side openings of the forward support, and positioned in relation to the horizontal slots by the distancers (20), and said volatilizing combination is powered by means of wires (21) by the electrical system of the fan which, through contacts (1T, 2T and 3T), energizes the thermal plate through switching (22), which when turned clockwise also drives the fan propellers at the selected speed (V1, V2, and V3), and when turned anticlockwise only drives the fan propellers at the selected speed (V1, V2, and V3).

2. Fan (2) **characterized in that** the fan (2) comprises the constructive arrangement according to claim 1.
